# FASCICULE DE BREVET EUROPEEN

(11) **EP 2 490 823 B1**
(45) Date de publication et mention de la délivrance du brevet: **17.12.2014**
(21) Numéro de dépôt: 10785482.0
(22) Date de dépôt: 20.10.2010
(51) Int. Cl.: B05B 17/06, B65D 83/28, A61M 15/00, A61M 15/08, B05B 11/00

(54) **TETE DE DISTRIBUTION POUR DISPOSITIF DE DISTRIBUTION DE PRODUIT FLUIDE**
SPENDERKOPF FÜR EINEN FLUIDSPENDER
DISPENSING HEAD FOR A FLUID DISPENSER

(30) Priorité: 20.10.2009 FR 0957337
(43) Date de publication de la demande: 29.08.2012
(73) Titulaire: Aptar France SAS, 27110 Le Neubourg (FR)
(72) Inventeur: PARDONGE, Jean-Marc, F-76520 Les Authieux Sur Port Saint Ouen (FR)
(74) Mandataire: CAPRI
(86) Numéro de dépôt international: PCT/FR2010/052235
(87) Numéro de publication internationale: WO 2011/048330

(56) Documents cités:
- DE-A1- 10 102 846
- JP-A- 8 332 425
- JP-A- 8 332 426
- US-A- 3 224 677
- US-A- 3 739 952
- US-A1- 2005 100 512

## Description

La présente invention concerne un dispositif de distribution de produit fluide comportant une tête de distribution.

Plus particulièrement, la présente invention concerne des dispositifs de distribution de produit fluide adaptés pour distribuer ou pulvériser des doses de principes actifs biologiques, tel que par exemple des protéines, des peptides, des hormones, des enzymes ou similaires. En particulier, cette distribution peut être réalisée sous forme de spray nasal. Les dispositifs de distribution de produit fluide utilisés jusqu'à ce jour dans l'industrie pharmaceutique comportent de manière connue généralement un réservoir sur lequel est monté une pompe ou une valve qui est actionnée au moyen d'un poussoir ou tête de distribution pourvue d'un orifice de pulvérisation. Ces dispositifs qui sont parfaitement adaptés pour distribuer du produit liquide classique dans l'industrie pharmaceutique, s'avèrent plus difficiles à utiliser pour distribuer des principes actifs biologiques tel que les protéines, peptides, hormones, enzymes ou similaires. En particulier, les caractéristiques mécaniques et fluidiques fournies par les pompes ou les valves, et notamment les pertes de charge, les niveaux de turbulence et les contraintes de cisaillement, se sont généralement avérées incompatibles avec la structure moléculaire tridimensionnelle desdits principes actifs biologiques. Il s'ensuit que ces principes actifs biologiques sont généralement détériorés lors de la distribution à travers des dispositifs de distribution classiques, en particulier dans les profils de pulvérisation.

Les documents JP 08332426 et JP 08332425 décivent des dispositifs de distribution munis de moyens de génération de vibration et le document US 2005/0100512 décrit une tête qui fournit par intermittence une distribution d'un produit fluide alimenté par une valve.

La présente invention a pour but de fournir un dispositif de distribution de produit fluide comportant une tête de distribution qui ne reproduit pas les inconvénients susmentionnés.

Plus particulièrement, la présente invention a pour but de fournir un dispositif de distribution adapté pour distribuer des principes actifs biologiques sans les détériorer de manière négative.

La présente invention a aussi pour but de fournir un tel dispositif de distribution de produit fluide adapté à fournir des performances de spray compatibles avec la nature des principes actifs biologiques tel que les protéines, les peptides, les hormones, les enzymes ou similaires.

La présente invention a également pour but de fournir un tel dispositif de distribution de produit fluide qui soit simple et peu coûteux à fabriquer et à assembler.

La présente invention a donc pour objet un dispositif de distribution de produit fluide , comportant un réservoir contenant du produit fluide, un organe de distribution, tel qu'une pompe ou une valve, monté sur ledit réservoir, et une tête de distribution montée sur ledit organe de distribution, ladite tête de distribution comprenant un corps creux, définissant un passage de produit fluide entre un orifice d'entrée, relié audit organe de distribution, et un orifice de pulvérisation, des moyens de génération de vibrations étant disposés dans ledit passage en amont dudit orifice de pulvérisation, lesdits moyens de génération de vibrations étant actionnés lors du passage du produit fluide pour finement pulvériser ledit produit fluide à travers ledit orifice de pulvérisation.

Avantageusement, lesdits moyens de génération de vibrations comportent un matériau piézoélectrique.

Selon un premier mode de réalisation avantageux, lesdits moyens de génération de vibrations comportent un pointeau vibrant comportant une pointe adaptée à obturer ledit orifice de pulvérisation dans une position de fermeture.

Avantageusement, ledit orifice d'entrée et ledit orifice de pulvérisation sont orientés axialement le long d'un axe longitudinal de ladite tête, ledit pointeau étant déplaçable et vibrant selon ledit axe longitudinal.

Avantageusement, en position de fermeture, ladite pointe coopère de manière étanche avec une membrane d'étanchéité disposée directement en amont dudit orifice de pulvérisation.

Avantageusement, ledit pointeau est fixé sur une plaque piézoélectrique.

Avantageusement, ledit pointeau est déplaçable axialement dans ladite tête entre une position de fermeture, dans laquelle il obture l'orifice de pulvérisation et un passage de produit fluide, définissant ainsi une chambre de pulvérisation, et une position ouverte, dans laquelle il ouvre ledit orifice de pulvérisation et ledit passage de produit fluide, permettant au produit fluide de passer dudit orifice d'entrée vers ledit orifice de pulvérisation.

Avantageusement, ledit passage de produit fluide est formé par une partie de plus grand diamètre réalisé à l'intérieur de ladite tête de distribution.

Selon un second mode de réalisation avantageux, lesdits moyens de génération de vibrations comportent une plaque vibrante disposée sur une paroi interne de ladite tête, le produit fluide impactant ladite plaque vibrante.

Avantageusement, ledit orifice de sortie est orienté environ perpendiculairement à la direction d'écoulement du produit fluide à travers l'orifice d'entrée, ladite plaque vibrante étant disposée sur une paroi interne inclinée, de préférence à environ 45°, de sorte que le jet de produit fluide impactant ladite plaque vibrante est fragmenté en spray et dirigé vers l'orifice de pulvérisation.

Avantageusement, ladite plaque vibrante forme une paroi d'une chambre de pulvérisation comportant une entrée de chambre reliée audit orifice d'entrée et pourvue dudit orifice de pulvérisation.

Selon un troisième mode de réalisation avantageux, lesdits moyens de génération de vibrations comportent une membrane vibrante définissant entre ladite membrane vibrante et l'orifice de pulvérisation une chambre de résonance.

Avantageusement, ladite chambre de résonance est sensiblement en forme de cylindre ayant une longueur et un diamètre inférieurs aux longueurs d'ondes de résonances de ladite membrane, ladite chambre de résonance formant un résonateur de Helmholtz.

Avantageusement, ladite membrane a sensiblement le même diamètre que ladite chambre de résonance, ladite tête comportant un passage pour le produit fluide s'étendant radialement à l'extérieur de ladite membrane.

Avantageusement, le spray de produit fluide pulvérisé à travers ledit orifice de pulvérisation comporte une taille de particule moyenne comprise entre 20 et 40 µm et/ou une vitesse de spray inférieure à 10 m/s, avantageusement inférieure à 5 m/s, de préférence environ 1 m/s, et/ou un angle de spray inférieur à 50°, avantageusement environ 30°.

Ces caractéristiques et avantages et d'autres de la présente invention apparaîtront plus clairement au cours de la description détaillée suivante de trois modes de réalisation de la présente invention, faite en référence aux dessins joints, donnés à titre d'exemples non limitatifs, et sur lesquels
la figure 1 est une vue schématique en section transversale d'un dispositif de distribution de produit fluide auquel la présente invention peut s'appliquer,
la figure 2 est une vue schématique de détail en section transversale d'une partie d'une tête de distribution de produit fluide selon un premier mode de réalisation de la présente invention, en position de fermeture,
la figure 3 est une vue similaire à celle de la figure 2, en position d'ouverture,
la figure 4 est une vue similaire à celle des figures 2 et 3, représentant un second mode de réalisation de la présente invention,
la figure 5 est une vue similaire aux figures 2, 3 et 4, représentant un troisième mode de réalisation de la présente invention.

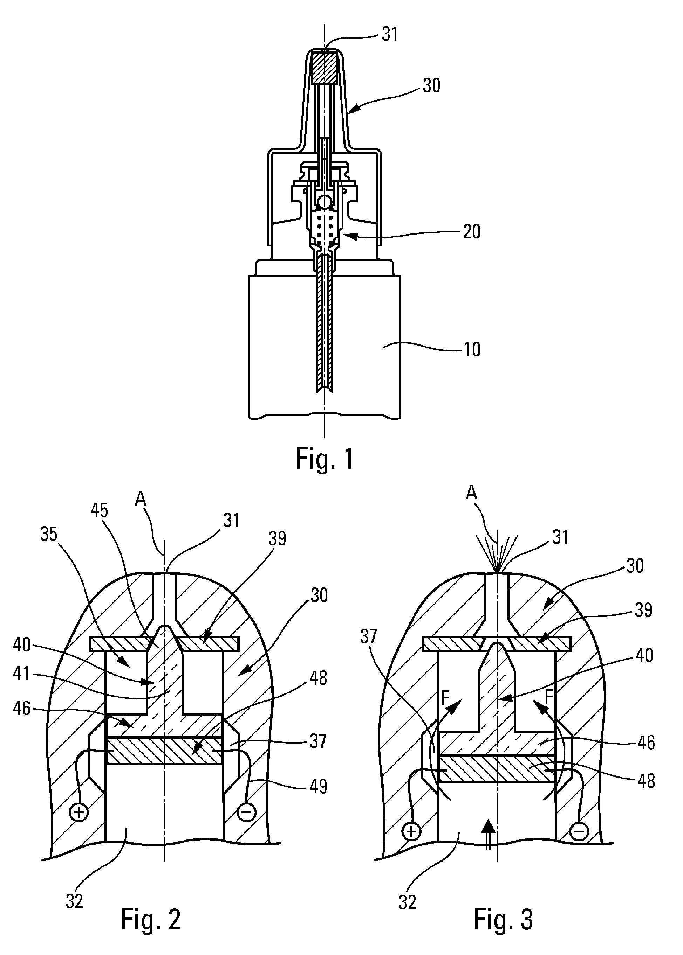

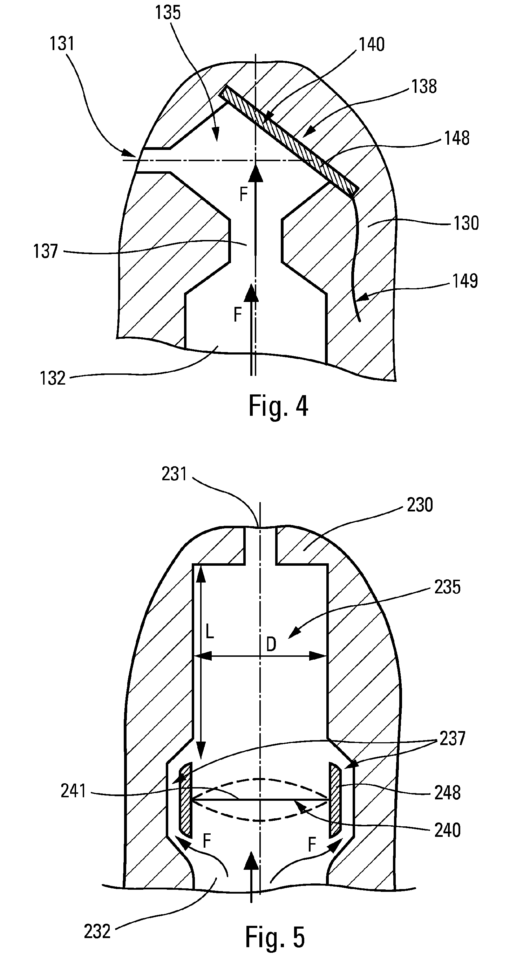

La figure 1 est une vue schématique d'un dispositif de distribution qui comporte un réservoir 10 contenant le produit fluide à distribuer, sur lequel est assemblé un organe de distribution, en l'occurrence une pompe 20, actionnée par un poussoir ou tête de distribution pourvu d'un orifice de pulvérisation 31. Dans l'exemple de la figure 1, la tête de distribution est de type nasal et comporte un corps 30 pourvu d'une extension axiale dont l'extrémité est pourvue de l'orifice de pulvérisation 31, ladite extension axiale étant destinée à pénétrer dans la narine de l'utilisateur. Bien entendu, la présente invention s'applique aussi à d'autres types de dispositifs de distribution de produit fluide. En particulier, l'organe de distribution 20 peut être une pompe d'un type différent, voire une valve. De même, la tête de distribution peut être de forme différente, avec notamment un orifice de pulvérisation 31 dirigé dans une direction autre que la direction axiale représentée sur la figure 1, et notamment dans une direction environ perpendiculaire à cette direction axiale, comme représenté sur la figure 4.

Dans un dispositif de distribution de produit fluide classique, la tête de distribution comporte en amont de l'orifice de pulvérisation 31 un profil de pulvérisation, généralement formé de canaux non radiaux reliant le canal d'expulsion de la tête à une chambre de tourbillonnement centrale disposée directement en amont de l'orifice de pulvérisation. Un tel profil de pulvérisation induit des contraintes de cisaillement importantes du produit fluide par succession de contractions/détentes, notamment au niveau des canaux non radiaux et de la chambre de tourbillonnement.

La présente invention prévoit de remplacer ce profil de pulvérisation par un système plus adapté à l'utilisation avec des principes actifs biologiques, tels que des protéines, des peptides, des hormones, des enzymes ou similaires, tout en garantissant une parfaite pulvérisation desdits principes actifs biologiques. En particulier, la présente invention a pour but de fournir les dispositifs de distribution de produit fluide capables de satisfaire un certain nombre de performances de spray, notamment au niveau de la taille des particules du spray, qui sont de préférence comprises entre 20 et 40 µm. Avantageusement, aucune particule n'aura une taille de particule inférieure à 10 µm. Par ailleurs, la vitesse de spray est de préférence inférieure à 10 m/s, et idéalement d'environ 1 m/s. L'angle de spray, c'est-à-dire l'angle à l'intérieur duquel se retrouveront les différentes particules du spray après la sortie à travers l'orifice de pulvérisation 31, est de préférence inférieur à 50°, et avantageusement environ de 30°. La combinaison de ces performances s'avère particulièrement adaptée à la distribution de principes actifs biologiques, dont les molécules ont des tailles tridimensionnelles importantes relativement fragiles et donc soumises à destruction dans les profils de pulvérisation classiques. Avantageusement, ces caractéristiques sont appliquées à des doses de 50 à 100 microlitres de produit fluide, et la tête nasale est de préférence insérée dans la narine sur une profondeur d'environ 1 centimètre. Bien entendu, ces valeurs ne sont pas limitatives mais seulement données à titre d'exemple.

Pour garantir l'obtention de ces caractéristiques ou performances de spray, la présente invention prévoit des moyens de génération de vibration 40, 140, 240 dans le corps creux 30, 130, 230 de la tête de distribution, lesdits moyens de génération de vibrations étant actionnés au moment du passage du produit fluide issu de l'organe de distribution 20, en l'occurrence de préférence une pompe mécanique, l'actionnement desdits moyens de génération de vibrations permettant de finement pulvériser ledit produit fluide à travers ledit orifice de pulvérisation 31, 131, 231. Ce sont donc les moyens de génération de vibrations qui subissent des contraintes haute fréquences qui permettent de pulvériser le produit fluide, celui-ci ne subissant plus les contraintes, notamment de cisaillement, des profils de pulvérisations classiques.

Les figures 2 et 3 représentent schématiquement un premier mode de réalisation de la présente invention. Dans ce premier mode de réalisation, les moyens de génération de vibrations 40 comportent un pointeau 41. Ce pointeau 41 est associé de préférence à une plaque piézoélectrique 48 reliée à une source d'alimentation 49, l'excitation de la plaque piézoélectrique 48 suscitant une vibration dudit pointeau 41. Le pointeau 41 comporte dans son extrémité aval une pointe 45 adaptée à obturer de manière étanche l'orifice de pulvérisation 31 de la tête de distribution lorsqu'il est en position de fermeture. De préférence, cette obturation en position de fermeture se fait par coopération avec une membrane 39 disposée directement en amont dudit orifice de pulvérisation 31, comme représenté sur les figures 2 et 3. Le pointeau 41 coulisse de préférence dans un canal sensiblement cylindrique formé à l'intérieur du corps 30 de la tête de distribution, ce canal s'étendant entre l'orifice d'entrée 32 et l'orifice de pulvérisation 31. De préférence, ces orifice d'entrée et de pulvérisation se retrouvent axialement le long d'un axe longitudinal A et ledit pointeau se déplace et vibre selon ledit même axe A. Avantageusement, le pointeau 41 comporte dans sa partie amont un disque 46 fixé sur ladite plaque piézoélectrique 48, et dont le diamètre extérieur est sensiblement égal au diamètre intérieur dudit cylindre dans lequel le pointeau se déplace. Ainsi, en position de fermeture, ledit pointeau 41 isole une chambre de pulvérisation 35, ladite pointe 45 du pointeau obturant de manière étanche l'orifice de pulvérisation 31, et ladite plaque arrière 46 dudit pointeau fermant un passage de produit fluide 37 réalisé dans le corps 30 de ladite tête de distribution. Comme visible sur les figure 2 et 3, ledit passage de produit fluide 37 est avantageusement réalisé dans une paroi latérale de ladite tête, par une partie de diamètre supérieur, de sorte qu'en position d'ouverture représenté sur la figure 3, le produit fluide peut s'écouler latéralement autour dudit pointeau 41, notamment autour de ladite plaque piézoélectrique 48 et dudit disque 46, comme symbolisé par les flèches F sur la figure 3. Dans cette position d'ouverture, la vibration du pointeau 41 provoquera la distribution de fines gouttelettes de spray à partir du produit fluide arrivant dans ladite chambre de pulvérisation 35, sans toutefois exercer des contraintes négatives sur le produit fluide, ce qui rend le dispositif adapté à la distribution de principes actifs biologiques.

La figure 4 représente un second mode de réalisation de la présente invention. Dans ce second mode de réalisation, les moyens de génération de vibrations 140 comportent une plaque vibrante 148 disposée sur une paroi interne du corps 130 de ladite tête de distribution. Le produit fluide issu de la pompe 20, de préférence sous forme de gel, vient impacter ladite plaque vibrante 148, et la vibration de cette plaque vibrante 148 provoquera la pulvérisation en fines gouttelettes de spray dudit produit fluide, qui sera alors distribué à travers ledit orifice de pulvérisation 131. Dans l'exemple de réalisation de la figure 4, l'orifice de pulvérisation est orienté environ perpendiculairement à l'axe central de la tête qui correspond à la direction de déplacement du produit fluide, notamment à travers l'orifice d'entrée 132, comme symbolisé par les flèches F. Dans cette mise en oeuvre, la plaque vibrante 148 est de préférence disposée selon un plan incliné, avantageusement à environ 45°, par rapport à la direction F, pour automatiquement et directement diriger les gouttelettes de produit fluide en direction de l'orifice de pulvérisation 131. La plaque vibrante 148 est de préférence formée par un matériau piézoélectrique, reliée à une alimentation électrique 149, qui n'est que très schématiquement représentée sur la figure 4, et qui peut être de toute forme appropriée. Ainsi, le jet de produit fluide qui pénètre dans la tête de distribution à travers l'orifice d'entrée 132 vient impacter la plaque vibrante 148 qui fragmente alors ce jet en fines gouttelettes de spray, sans toutefois soumettre ledit produit fluide à des contraintes de cisaillement et/ou des turbulences trop importantes susceptibles d'être incompatibles avec la distribution de produits de principes actifs biologiques. Avantageusement, comme représenté sur la figure 4, une chambre de pulvérisation 135 est formée directement en amont de l'orifice de pulvérisation 131, ladite plaque vibrante 148 formant une paroi de ladite chambre de pulvérisation 135. Ladite chambre comporte également un passage d'entrée de produit fluide 137, formé de préférence par une partie de diamètre réduit dans le corps 130 de la tête de distribution. La présence de cette chambre entre la plaque vibrante 148 et l'orifice de pulvérisation 131 favorise la formation du spray.

La figure 5 représente un troisième mode de réalisation de la présente invention. Dans ce troisième mode de réalisation, les moyens de génération de vibrations 240 comportent une membrane vibrante 241 disposée à l'intérieure du corps 230 de ladite tête de distribution. Ce troisième mode de réalisation prévoit en fait de former un résonateur de Helmholtz directement en amont de l'orifice de pulvérisation 231, afin de garantir la pulvérisation du jet de liquide provenant de la pompe. Ce résonateur de Helmholtz est avantageusement formé par une chambre de résonance 235 sensiblement cylindrique ayant une longueur L et un diamètre D. Ladite chambre de résonance 235 débouche de son côté aval dans l'orifice de pulvérisation 231 et comporte, du côté opposé, ladite membrane vibrante 241. De préférence le diamètre de cette membrane vibrante est sensiblement égal au diamètre D de la chambre de résonance 235. Les dimensions de longueur L et de diamètre D du résonateur sont bien entendu choisies de manière à être inférieures aux longueurs d'ondes mises en jeu, notamment celles de résonance de la membrane vibrante 241, comme cela est typique pour un résonateur de Helmholtz. Ainsi, ce résonateur de Helmholtz, notamment le volume de la chambre de résonance 235, a le rôle d'un ressort hydraulique qui pompe le liquide et contribue au fractionnement du spray au niveau de l'orifice de pulvérisation 231, sans toutefois imposer des contraintes de cisaillement ou d'autres contraintes mécaniques et physiques trop importantes sur le produit fluide, rendant ce dispositif particulièrement adapté à la distribution de principes actifs biologiques. Comme représenté schématiquement sur la figure 5, la tête de distribution comporte une partie de plus grand diamètre 237 formant un passage pour le produit fluide radialement à l'extérieur de ladite membrane vibrante 241. L'écoulement du produit fluide à cet endroit est symbolisé par les flèches F.

Bien que la présente invention a été décrite et représentée en référence à trois modes de réalisation de celle-ci, il est entendu qu'elle n'est pas limitée à ces modes de réalisation, mais qu'au contraire un homme du métier peut y apporter toutes modifications utiles sans sortir du cadre de la présente invention, tel que définit par les revendications annexées.

## Revendications

1. Dispositif de distribution de produit fluide, comportant un réservoir (10) contenant du produit fluide, un organe de distribution (20), tel qu'une pompe ou une valve, monté sur ledit réservoir (10), et une tête de distribution montée sur ledit organe de distribution (20), ladite tête de distribution comprenant un corps creux (30, 130, 230), définissant un passage de produit fluide entre un orifice d'entrée (32, 132, 232), relié audit organe de distribution (20), et un orifice de pulvérisation (31, 131, 231), **caractérisé en ce que** des moyens de génération de vibrations (40, 140, 240) sont disposés dans ledit passage en amont dudit orifice de pulvérisation (31, 131, 231), lesdits moyens de génération de vibrations (40, 140, 240) étant actionnés lors du passage du produit fluide pour finement pulvériser ledit produit fluide à travers ledit orifice de pulvérisation (31, 131, 231).

2. Dispositif selon la revendication 1, dans lequel lesdits moyens de génération de vibrations (40, 140, 240) comportent un matériau piézoélectrique.

3. Dispositif selon la revendication 1 ou 2, dans lequel lesdits moyens de génération de vibrations (40) comportent un pointeau vibrant (41) comportant une pointe (45) adaptée à obturer ledit orifice de pulvérisation (31) dans une position de fermeture.

4. Dispositif selon la revendication 3, dans lequel ledit orifice d'entrée (32) et ledit orifice de pulvérisation (31) sont orientés axialement le long d'un axe longitudinal (A) de ladite tête, ledit pointeau (41) étant déplaçable et vibrant selon ledit axe longitudinal (A).

5. Dispositif selon la revendication 3 ou 4, dans lequel, en position de fermeture, ladite pointe (45) coopère de manière étanche avec une membrane d'étanchéité (39) disposée directement en amont dudit orifice de pulvérisation (31).

6. Dispositif selon l'une quelconque des revendications 3 à 5, dans lequel ledit pointeau (41) est fixé sur une plaque piézoélectrique (48).

7. Dispositif selon l'une quelconque des revendications 3 à 6, dans lequel ledit pointeau (41) est déplaçable axialement dans ladite tête entre une position de fermeture, dans laquelle il obture l'orifice de pulvérisation (31) et un passage de produit fluide (37), définissant ainsi une chambre de pulvérisation (35), et une position ouverte, dans laquelle il ouvre ledit orifice de pulvérisation (31) et ledit passage de produit fluide (37), permettant au produit fluide de passer dudit orifice d'entrée (32) vers ledit orifice de pulvérisation (31).

8. Dispositif selon la revendication 7, dans lequel ledit passage de produit fluide (37) est formé par une partie de plus grand diamètre réalisé à l'intérieur de ladite tête de distribution.

9. Dispositif selon la revendication 1 ou 2, dans lequel lesdits moyens de génération de vibrations (140) comportent une plaque vibrante (148) disposée sur une paroi interne de ladite tête, le produit fluide impactant ladite plaque vibrante (148).

10. Dispositif selon la revendication 9, dans lequel ledit orifice de sortie (131) est orienté environ perpendiculairement à la direction d'écoulement du produit fluide (F) à travers l'orifice d'entrée (132), ladite plaque vibrante étant disposée sur une paroi interne inclinée (138), de préférence à environ 45°, de sorte que le jet de produit fluide impactant ladite plaque vibrante (148) est fragmenté en spray et dirigé vers l'orifice de pulvérisation (131).

11. Dispositif selon la revendication 9 ou 10, dans lequel ladite plaque vibrante (148) forme une paroi d'une chambre de pulvérisation (135) comportant une entrée de chambre (137) reliée audit orifice d'entrée (132) et pourvue dudit orifice de pulvérisation (131).

12. Dispositif selon la revendication 1 ou 2, dans lequel lesdits moyens de génération de vibrations (240) comportent une membrane vibrante (241) définissant entre ladite membrane vibrante (241) et l'orifice de pulvérisation (231) une chambre de résonance (235).

13. Dispositif selon la revendication 12, dans lequel ladite chambre de résonance (235) est sensiblement en forme de cylindre ayant une longueur (L) et un diamètre (D) inférieurs aux longueurs d'ondes de résonances de ladite membrane (241), ladite chambre de résonance (235) formant un résonateur de Helmholtz.

14. Dispositif selon la revendication 13, dans lequel ladite membrane (241) a sensiblement le même diamètre (D) que ladite chambre de résonance (235), ladite tête comportant un passage pour le produit fluide (237) s'étendant radialement à l'extérieur de ladite membrane (241).

15. Dispositif selon l'une quelconque des revendications précédentes, dans lequel le spray de produit fluide pulvérisé à travers ledit orifice de pulvérisation (31, 131, 231) comporte une taille de particule moyenne comprise entre 20 et 40 µm et/ou une vitesse de spray inférieure à 10 m/s, avantageusement inférieure à 5 m/s, de préférence environ 1 m/s, et/ou un angle de spray inférieur à 50°, avantageusement environ 30°.

## Patentansprüche

1. Ausgabevorrichtung für ein fluides Produkt, aufweisend einen Behälter (10), der ein fluides Produkt enthält, eine Ausgabeeinrichtung (20), wie eine Pumpe oder ein Ventil, die auf dem Behälter (10) montiert ist, und einen Ausgabekopf, der auf der Ausgabeeinrichtung (20) montiert ist, wobei der Ausgabekopf einen Hohlkörper (30, 130, 230) aufweist, der einen Durchgang für fluides Produkt zwischen einer mit der Ausgabeeinrichtung (20) verbundenen Eintrittsöffnung (32, 132, 232) und einer Zerstäubungsöffnung (31, 131, 231) definiert, **dadurch gekennzeichnet, dass** Vibrationserzeugungsmittel (40, 140, 240) in dem Durchgang stromaufwärts der Zerstäubungsöffnung (31, 131, 231) angeordnet sind, wobei die Vibrationserzeugungsmittel (40, 140, 240) beim Durchgang des fluiden Produktes betätigt werden, um das fluide Produkt durch die Zerstäubungsöffnung (31, 131, 231) fein zu zerstäuben.

2. Vorrichtung nach Anspruch 1, wobei die Vibrationserzeugungsmittel (40, 140, 240) ein piezoelektrisches Material umfassen.

3. Vorrichtung nach Anspruch 1 oder 2, wobei die Vibrationserzeugungsmittel (40) eine vibrierende Düsennadel (41) aufweisen, die eine Spitze (45) aufweist, die geeignet ist, die Zerstäubungsöffnung (31) in einer Schließposition zu verschließen.

4. Vorrichtung nach Anspruch 3, wobei die Eintrittsöffnung (32) und die Zerstäubungsöffnung (31) axial entlang einer Längsachse (A) des Kopfes ausgerichtet sind, wobei die Düsennadel (41) entlang der Längsachse (A) verschiebbar und vibrierend ist.

5. Vorrichtung nach Anspruch 3 oder 4, wobei die Spitze (45) in der Schließposition dichtend mit einer Dichtigkeitsmembran (39) zusammenwirkt, die direkt stromaufwärts der Zerstäubungsöffnung (31) angeordnet ist.

6. Vorrichtung nach einem der Ansprüche 3 bis 5, wobei die Düsennadel (41) auf einer piezoelektrischen Platte (48) befestigt ist.

7. Vorrichtung nach einem der Ansprüche 3 bis 6, wobei die Düsennadel (41) axial in dem Kopf zwischen einer Schließposition, in der sie die Zerstäubungsöffnung (31) und einen Durchgang für ein fluides Produkt (37) verschließt, wodurch sie eine Zerstäubungskammer (35) definiert, und einer geöffneten Position, in der sie die Zerstäubungsöffnung (31) und den Durchgang für ein fluides Produkt (37) öffnet, wodurch sie dem fluiden Produkt den Durchgang von der Eintrittsöffnung (32) zur Zerstäubungsöffnung (31) ermöglicht, verschiebbar ist.

8. Vorrichtung nach Anspruch 7, wobei der Durchgang für ein fluides Produkt (37) durch einen Teil von größerem Durchmesser gebildet wird, der im Inneren des Ausgabekopfes ausgebildet ist.

9. Vorrichtung nach Anspruch 1 oder 2, wobei die Vibrationserzeugungsmittel (140) eine Vibrationsplatte (148) aufweisen, die an einer Innenwand des Kopfes angeordnet ist, wobei das fluide Produkt auf die Vibrationsplatte (148) auftrifft.

10. Vorrichtung nach Anspruch 9, wobei die Austrittsöffnung (131) in etwa senkrecht zur Fließrichtung des fluiden Produktes (F) durch die Eintrittsöffnung (132) ausgerichtet ist, wobei die Vibrationsplatte an einer Innenwand (138) angeordnet ist, die geneigt ist, vorzugsweise um etwa 45°, so dass der Strahl des fluiden Produktes, der auf die Vibrationsplatte (148) trifft, in ein Spray geteilt und zur Zerstäubungsöffnung (131) gelenkt wird.

11. Vorrichtung nach Anspruch 9 oder 10, wobei die Vibrationsplatte (148) eine Wand einer Zerstäubungskammer (135) bildet, die einen Kammereingang (137) aufweist, der mit der Eintrittsöffnung (132) verbunden und mit der Zerstäubungsöffnung (131) versehen ist.

12. Vorrichtung nach Anspruch 1 oder 2, wobei die Vibrationserzeugungsmittel (240) eine Vibrationsmembran (241) aufweisen, die zwischen der Vibrationsmembran (241) und der Zerstäubungsöffnung (231) eine Resonanzkammer (235) bildet.

13. Vorrichtung nach Anspruch 12, wobei die Resonanzkammer (235) im Wesentlichen eine zylindrische Form mit einer Länge (L) und einem Durchmesser (D) aufweist, die kleiner als die Längen der Resonanzwellen der Membran (241) sind, wobei die Resonanzkammer (235) einen Helmholtz-Resonator bildet.

14. Vorrichtung nach Anspruch 13, wobei die Membran (241) im Wesentlichen den gleichen Durchmesser (D) aufweist wie die Resonanzkammer (235), wobei der Kopf einen Durchgang für das fluide Produkt (237) aufweist, der sich radial nach außerhalb der Membran (241) erstreckt.

15. Vorrichtung nach einem der vorhergehenden Ansprüche, wobei das Spray von fluidem Produkt, das durch die Zerstäubungsöffnung (31, 131, 231) zerstäubt wird, eine mittlere Teilchengröße zwischen 20 und 40 µm und/oder eine Spraygeschwindigkeit von weniger als 10 m/s, vorteilhafterweise weniger als 5 m/s, vorzugsweise etwa 1 m/s, und/oder einen Spraywinkel von weniger als 50°, vorteilhafterweise etwa 30°, aufweist.

## Claims

1. A fluid dispenser device comprising: a reservoir (10) containing fluid; a dispenser member (20), such as a pump or a valve, that is mounted on said reservoir (10); and a dispenser head that is mounted on said dispenser member (20), said dispenser head including a hollow body (30, 130, 230) that defines a fluid passage between an inlet orifice (32, 132, 232), connected to said dispenser member (20), and a spray orifice (31, 131, 231); the device being **characterized in that** vibration generator means (40, 140, 240) are arranged in said passage upstream from said spray orifice (31, 131, 231), said vibration generator means (40, 140, 240) being actuated while fluid is passing so as to spray said fluid finely through said spray orifice (31, 131, 231).

2. A device according to claim 1, wherein said vibration generator means (40, 140, 240) include a piezoelectric material.

3. A device according to claim 1 or claim 2, wherein said vibration generator means (40) include a vibrating pin (41) that includes a tip (45) that is adapted to plug said spray orifice (31) in a closed position.

4. A device according to claim 3, wherein said inlet orifice (32) and said spray orifice (31) are oriented axially along a longitudinal axis (A) of said head, said pin (41) being movable and vibrating along said longitudinal axis (A).

5. A device according to claim 3 or claim 4, wherein, in the closed position, said tip (45) co-operates in leaktight manner with a sealing diaphragm (39) that is arranged directly upstream from said spray orifice (31).

6. A device according to any one of claims 3 to 5, wherein said pin (41) is fastened on a piezoelectric plate (48).

7. A device according to any one of claims 3 to 6, wherein said pin (41) is axially movable in said head between a closed position in which it plugs the spray orifice (31) and a fluid passage (37), thereby defining a spray chamber (35), and an open position in which it opens said spray orifice (31) and said fluid passage (37), enabling the fluid to pass from said inlet orifice (32) to said spray orifice (31).

8. A device according to claim 7, wherein said fluid passage (37) is formed by a portion of greater diameter formed inside said dispenser head.

9. A device according to claim 1 or claim 2, wherein said vibration generator means (140) comprise a vibrating plate (148) that is arranged on an inside wall of said head, the fluid impacting said vibrating plate (148).

10. A device according to claim 9, wherein said outlet orifice (131) is oriented approximately perpendicularly to the direction of flow of the fluid (F) through the inlet orifice (132), said vibrating plate being arranged on an inside wall (138) that slopes, preferably at about 45°, so that the jet of fluid impacting said vibrating plate (148) is fragmented into spray and directed towards the spray orifice (131).

11. A device according to claim 9 or claim 10, wherein said vibrating plate (148) forms a wall of a spray chamber (135) that includes a chamber inlet (137) connected to said inlet orifice (132), and that is provided with said spray orifice (131).

12. A device according to claim 1 or claim 2, wherein said vibration generator means (240) comprise a vibrating diaphragm (241) that defines a resonance chamber (235) between said vibrating diaphragm (241) and the spray orifice (231).

13. A device according to claim 12, wherein said resonance chamber (235) is substantially in the shape of a cylinder having a length (L) and a diameter (D) that are shorter than the resonance wavelengths of said diaphragm (241), said resonance chamber (235) forming a Helmholtz resonator.

14. A device according to claim 13, wherein said diaphragm (241) has substantially the same diameter (D) as said resonance chamber (235), said head including a passage (237) for the fluid, which passage extends radially outside said diaphragm (241).

15. A device according to any preceding claim, wherein the fluid spray that is sprayed through said spray orifice (31, 131, 231) has a mean particle size lying in the range 20 µm to 40 µm, and/or a spray speed that is less than 10 m/s, advantageously less than 5 m/s, preferably about 1 m/s, and/or a spray angle that is less than 50°, advantageously about 30°.
